(19) Europäisches Patentamt
European Patent Office
Office européen des brevets

(11) Publication number: **0 066 325**
**B1**

(12) **EUROPEAN PATENT SPECIFICATION**

(45) Date of publication of patent specification: **17.07.85**

(51) Int. Cl.$^4$: **C 07 C 85/24** // C07C85/26

(21) Application number: **82200596.3**

(22) Date of filing: **14.05.82**

(54) **Process for the preparation of aniline derivatives.**

(30) Priority: **28.05.81 GB 8116386**

(43) Date of publication of application:
**08.12.82 Bulletin 82/49**

(45) Publication of the grant of the patent:
**17.07.85 Bulletin 85/29**

(84) Designated Contracting States:
**BE CH DE FR GB IT LI NL SE**

(56) References cited:
**GB-A-2 064 537**
**US-A-2 285 243**

**J. AM. CHEM. SOC., vol.61, May 1939, Washington D.C. (US) W.S. CALCOTT et al.: "Hydrofluoric acid as a condensing agent. II. Nuclear alkylations in the presence of hydrofluoric acid", pages 1010-1015**

**G.A.OLAH: "FRIEDEL-CRAFTS AND RELATED REACTIONS", vol.II, part 1, Interscience publishers, 1964, New York (US), pages 433-437**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Scholes, Gary**
**Badhuisweg 3**
**NL-1031 CM Amsterdam (NL)**
Inventor: **Van Helden, Robert**
**Badhuisweg 3**
**NL-1031 CM Amsterdam (NL)**

(74) Representative: **Hunter, Keith Roger Ian et al**
**4 York Road**
**London SE1 7NA (GB)**

(56) References cited:
**CHEMICAL ABSTRACTS, vol.66, no.21, May 22, 1967, abstract no.94439n, Columbus, Ohio (US) V.G. CHEKHUTA et al.: "Toluidine ring alkylation with isopropyl ether"**

**EP 0 066 325 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the preparation of aniline derivatives.

It is well known that aromatic compounds can be alkylated in the aromatic nucleus by reaction with various alkylating agents in the presence of a protonic acid or a Lewis acid as catalyst. This is the Friedel-Crafts reaction. A very wide range of acidic catalysts have been used to prepare a wide variety of alkylsubstituted benzene derivatives.

Such reactions are of great importance and of wide applicability. They can however suffer from two principle disadvantages. First, it can be rather difficult to prevent di-alkylation of the benzene nucleus, since the first product formed, the mono-alkylated product, tends to be more reactive than the starting material. Thus it is often necessary to conduct the reaction in such a way that only a relatively small proportion of the aromatic starting material reacts, and then to separate out the product or products formed, and re-cycle the starting material. On an industrial scale, such procedures can be very costly.

Secondly, when using certain substituted benzenes as starting materials, alkyl substitution can lead to two or more positional isomers. In some cases, one isomer is formed in large excess over the other possible isomers; in many cases however the reaction is relatively unspecific, and mixtures of some or all of the possible isomers are formed.

German Auslegeschrift No. 1,051,271, describes a Friedel-Crafts process for the nuclear alkylation of anilines. The major products produced are mono- or di-alkyl anilines with the alkyl group or groups *ortho* to the amino group. In addition, N-alkyl products are often produced. When using 2-methylaniline and propene as starting materials, the products obtained were 2-methyl-6-isopropylaniline and a large proportion of diisopropyl 2-methylanilines. When using 4-methylaniline and propene as starting materials, the only product isolated was 2,6-diisopropyl-4-methylaniline.

No process capable of commercial application is known to the Applicants whereby 3,4-dialkylanilines or N-alkyl derivatives thereof can be prepared by nuclear alkylation with reasonable selectivity and yield to the desired product. It is an object of the present invention to provide such a process.

The invention therefore provides a process for the preparation of a compound of the general formula

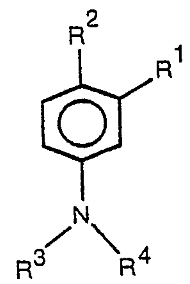

(I)

in which each of $R^1$ and $R^2$ independently represents an alkyl group and each of $R^3$ and $R^4$ independently represents a hydrogen atom or an alkyl group, which comprises reacting a compound of the general formula

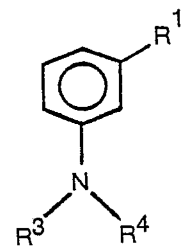

(II)

in which $R^1$, $R^3$ and $R^4$ have the meanings given above, with an alkylating agent selected from an alkene, an alkyl halide, an alkanol, an alkyl ester of a carboxylic acid, or a dialkyl ether, the reaction being conducted in the liquid phase in the presence of hydrogen fluoride.

Any alkyl group represented by $R^1$, $R^2$, $R^3$ or $R^4$ preferably has up to 6, especially up to 4, carbon atoms. Preferably $R^1$ represents a methyl group. Preferably $R^2$ represents an isopropyl group. Preferably both $R^3$ and $R^4$ represent hydrogen atoms.

An alkene, alkanol, alkyl halide, alkyl ester of a carboxylic acid or dialkyl ether may be used in the process of the present invention, the particular choice depending of course on the desired alkyl group $R^2$. As is normal in Friedel-Crafts reactions, the alkyl group $R^2$ introduced into the aniline nucleus may in some cases be a different group from an alkyl group present in the alkylating agent. Usually, products in which $R^2$ is a tertiary or secondary group rather than a primary group predominate when $R^2$ has 3 or more carbon atoms. For example, an isopropyl group $R^2$ can be introduced by the use of either isopropyl chloride or n-propyl chloride as alkylating agent: under the reaction conditions, the same transient intermediate is formed and leads mainly to isopropylation of the aniline nucleus.

In general, the use of an alkene or an alkyl halide is preferred in the process according to the invention. For reasons of cost, an alkyl chloride is usually the preferred alkyl halide. The use of alkanols produces an efficient reaction, but this reaction generates water, as does the use of dialkyl ethers. While this does not affect the course of the reaction, it may cause handling difficulties since water-containing HF is more corrosive than anhydrous HF. For this same reason, all reagents used in the process according to the invention should preferably be relatively dry, although the presence of the amounts of water normally found in commercial reagents presents no problem.

Thus when preparing a compound of the general formula I in which $R^2$ represents an isopropyl group, the alkylating agent is preferably a 1- or 2-halopropane for example 2-chloropropane, or, especially, propene.

Whatever alkylating agent is selected, it is in general preferred to conduct the reaction with incomplete conversion of the aniline to products. In this way the production of compounds containing two $R^2$ groups is minimised. Thus preferably the molar ratio of aniline to alkylating agent is from 1:1 to 5:1, especially in the range of from 1.1:1 to 3:1. However, when using an alkene as alkylating agent, polymerisation may occur as a rapid side-reaction, reducing the quantity of alkene available for use in the alkylation reaction; such polymerisation is especially rapid at relatively high temperatures. Thus when carrying out the reaction as a batch process, it is usually preferred to use an excess of alkene over aniline to ensure an adequate degree of alkylation. However, a preferred method of utilising an alkene is to meter in the alkene gradually during the course of the reaction, until the desired degree of conversion of the aniline is obtained. This method of operation can of course also be used for alkylating agents other than alkenes if desired.

It is believed, but not confirmed, that whatever the alkylating agent used, the first step is the formation of an alkyl fluoride. As compounds of this type are usually gaseous, the process according to the invention is suitably carried out in a contained system to prevent escape of material from the reaction mixture. If desired, the reaction may be carried out under a pressure in excess of atmospheric pressure, for example under an atmosphere of an inert gas, such as nitrogen. Excess pressures of up to 20 bars, preferably up to 10 bars, may for example be used. Higher pressures are of course possible, but are generally undesirable for economic reasons.

The boiling point of hydrogen fluoride at atmospheric pressure if 19°C, and so in general the use of pressures greater than atmospheric are necessary of it is desired to conduct the reaction at temperatures higher than this. The reaction temperature is not critical. Suitably the reaction is carried out at a temperature in the range of from −30 to 80°C, for example −30 to 40°C, especially 15 to 40°C.

For handling reasons, it is generally preferred to use excess liquid HF as a solvent for the reaction, for example at least 15 moles of HF per mole of aniline II, and preferably the aniline compound of the general formula II, the alkylating agent and the liquid HF are the only species present in the reaction mixture at the beginning of the reaction. In such a case, the mixture can be worked up after the reaction in a very simple manner, involving evaporation of the HF. However, other species may be present if desired. For example, an inert solvent or diluent may be present; aliphatic hydrocarbons, for example pentane, hexane or octane, and inert cyclic ethers such as tetrahydrofuran, are examples of suitable compounds. Other possible additives to the reaction mixture include certain non-metal halides, such as boron trifluoride or antimony pentafluoride, and strong inorganic acids, for example monofluorosulphuric acid. Such compounds tend to increase the acid strength of the reaction mixture, and may lower the temperature at which a given rate of reaction is attainable, or increase the rate of reaction at a given temperature.

In general, the compound of the general formula I is of course initially present in the reaction mixture in the form of its hydrofluoride salt. This may be isolated as such, or converted by known methods into the free aniline or any other acid addition salt, as desired. A convenient method of isolation is as the hydrochloride salt. The hydrochloride salt of 3-methyl-4-isopropylaniline can be precipitated out from an organic solution containing other isomeric methylisopropylanilines, by bubbling an equivalent amount of HCl to the 3-methyl-4-isopropylaniline, through the solution.

The process according to the invention is capable of producing the desired compound in which the $R^2$ group is *para* to the $-NR^3R^4$ group, in high selectivity compared with the other possible isomers and compared to compounds containing two $R^2$ groups. As shown in comparative Examples 5 to 8 herein, the use of various acidic materials known to be generally useful as catalysts in Friedel-Crafts reactions leads to a far less selective reaction for a given level of conversion of aniline starting material, or to no reaction at all.

The compound of the general formula I in which $R^1$ is methyl $R^2$ is isopropyl and $R^3$ and $R^4$ are both hydrogen, can be readily converted by methods analogous to known methods into the compound 3-(3-methyl-4-isopropylphenyl)-1,1-dimethylurea, which is an extremely potent selective herbicide for the control of weeds in cereal crops. Suitable processes for such conversion include reaction of the aniline with a dimethylcarbamoyl halide, or reaction of the aniline with phosgene followed by reaction with dimethylamine.

The following Examples illustrate the invention. Analyses were carried out by NMR and/or by gas-liquid chromatography. All percentage yields given are GLC area percentages.

Examples 1 to 4

0.2 mol 3-methylaniline were dissolved in 100 mls liquid hydrogen fluoride in a plastic container. The resulting solution was then sucked into an evacuated autoclave made of Hastelloy C (Trade Mark). The autoclave was pressurised to 4 bars with nitrogen, propene was then introduced, and the reaction mixture was maintained at the stated temperature for the stated reaction time. The hydrogen fluoride was then evaporated off and the products were analysed. The results are given in Table I below (3—MA = 3-methyl aniline; 3—M—4IA = 3-methyl-4-isopropylaniline).

TABLE I

| Example No. | Propene added (moles) | Temp °C | Reaction time (hours) | Conversion of 3—MA to products (%) | Product 3—M—4IA | distribution (%) diisopropyl compounds |
|---|---|---|---|---|---|---|
| 1 | 0.5 | 10 | 4 | 15 | 85 | 0 |
| 2 | 0.65 | 10 | 24 | 56 | 77 | 11 |
| 3 | 0.90 | 13 | 48 | 75 | 71 | 13 |
| 4 | 0.20 | 0 | 4 | 20 | 81 | 2 |

Comparative Experiments

A series of experiments were carried out on the alkylation of 3-methylaniline using several Friedel-Crafts catalysts, under conditions generally regarded as suitable for those catalysts.

Example 5 (comparison)

The general method of Examples 1 to 4 was repeated, except that 1g 3-methylaniline was dissolved in 3g 90% sulphuric acid. Propene was added to a pressure of 5 bars. After conducting the reaction at room temperature for 12 hours, analysis of the products showed that 53% of the starting material had been converted to products; 62% of the products was 3-methyl-4-isopropylaniline, and 27% of the products was diisopropyl compounds.

Example 6 (comparison)

The method of Example 5 was repeated, except that the propene was replaced by an equivalent amount of isopropanol and the reaction temperature was 80°C. At a conversion of 3-methylaniline to products of 60%, 60% of the products was the desired compound 3-methyl-4-isopropylaniline. Exactly the same results were obtained when a third of the sulphuric acid was replaced by the very strong acid, molybdic acid, $H_2MO_4$.

Example 7 (comparison)

In a continuous flow system consisting of a column filled with an active acidic clay catalyst $K_{106}$ (Trade Mark, Degussa), were passed 3-methylaniline (LHSV = 0.67 $h^{-1}$) and propene (GHSV = 4.5 × $10^2$ $h^{-1}$) at a temperature of 350°C and a total pressure of 5 bar. At 14% conversion of the aniline the product distribution was 2-isopropyl-5-methylaniline (65%m), 3-methyl-4-isopropylaniline (15%m), N-isopropyl-3-methylaniline (4%m), the rest being tarry products.

Replacement of the active clay catalyst by the acidic ion-exchange resin Nafion-H (Trade Mark, Dupont) a perfluorosulphonic acid/polystyrene resin, resulted only in tar formation at 170°C.

Example 8 (comparison)

Molybdenum carbonyl ($M_0(CO)_6$, 0.15 mol) was dissolved in cyclohexane (120 ml) and 3-methylaniline (0.1 mol) was added. The reaction mixture was heated under reflux for 2 hours. Analysis of the products showed that very little of the aniline had been converted to products.

Examples 9 to 17

A 250 ml autoclave was evacuated, and liquid hydrogen fluoride was introduced. Over a period of 20 minutes, the desired quantity of 3-methylaniline was pumped in, with cooling, followed by any other reaction components. Finally, the resulting mixture was warmed to the required reaction temperature and the alkylating agent was added gradually.

The mixture was stirred for the required reaction time (often, for convenience, overnight — 15½ hours) and thereafter the HF was evaporated off and the products analysed by GLC.

The results are given in Table II below.

TABLE II

| Example No. | HF (mls) | 3—MA (moles) | Other Additives (mls) | Alkylating Agent (moles) | Temp. °C | Reaction time (hrs) | Conversion of 3—MA to products (%) | Product 3—M—4IA (%) | distribution diisopropyl compounds (%) |
|---|---|---|---|---|---|---|---|---|---|
| 9 | 100 | 0.2 | — | propene (1.0) | 60 | 15½ | 51 | 71 | 15 |
| 10 | 100 | 0.2 | — | propene (1.0) | 30 | 15½ | 69 | 73 | 16 |
| 11 | 100 | 0.2 | — | isopropyl chloride (0.22) | 75 | 2 | 25 | 75 | 6 |
| 12 | 100 | 0.2 | — | isopropyl alcohol (0.26) | 60 | 5 | 21 | 74 | 10 |
| 13 | 100 | 0.2 | — | diisopropyl ether (0.11) | 60 | 2 | 20 | 73 | 8 |
| 14 | 90 | 0.2 | $FSO_3H$ (10) | propene (1.0) | 30 | 15½ | 77 | 66 | 23 |
| 15 | 100 | 0.2 | n-octane (9) | propene (0.2) | 15 | 15½ | 26 | 78 | 10 |
| 16 | 100 | 0.2 | n-octane (18) | propene (0.2) | 30 | 15½ | 46 | 79 | 15 |
| 17 | 100 | 0.2 | n-octane (18) | propene (0.2) | 60 | 15½ | 25 | 78 | 10 |

Example 18

The crude product obtained as in Examples 1 to 4 and 9 to 17 can be distilled to effect separation of the various components, but in some fractions isolated, separation is not always complete. This Example illustrates the separation of pure 3-methyl-4-isopropylaniline in the form of its HCl salt from a distillation fraction relatively rich in other anilines.

The fraction had the following composition:

| | |
|---|---|
| 3-methyl-4-isopropylaniline | 50.0% w |
| other methyl isopropylanilines | 45.0% w |
| methyl diisopropylanilines | 1.5% w |
| 3-methylaniline | 3.5% w |

149 g of this mixture were dissolved in toluene (275 ml) and a total of 27 g of hydrogen chloride gas was bubbled into the mixture over 20 minutes. Ice-cooling was provided as a result of which the temperature was in the range of 25—45°C. The mixture was then cooled to 15°C for 30 minutes. The precipitate was then filtered off, washed with cold toluene and then with hexane, and dried under vacuum at 50°C.

98 g of crystals were obtained, and shown by GLC to consist of the hydrochloride salt of 3-methyl-4-isopropylaniline of purity at least 99%.

**Claims**

1. A process for the preparation of a compound of the general formula

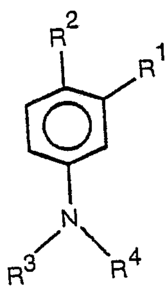

(I)

in which each of $R^1$ and $R^2$ independently represents an alkyl group and each of $R^3$ and $R^4$ independently represents a hydrogen atom or an alkyl group, which comprises reacting a compound of the general formula

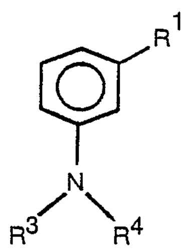

(II)

in which $R^1$, $R^3$ and $R^4$ have the meanings given above, with an alkylating agent selected from an alkene, an alkyl halide, an alkanol, an alkyl ester of a carboxylic acid, or a dialkyl ether, characterised in that the reaction being conducted in the liquid phase in the presence of hydrogen fluoride.

2. A process as claimed in claim 1, in which the alkylating agent is an alkene or an alkyl halide.

3. A process as claimed in either claim 1 or claim 2, in which the alkylating agent is added gradually to the reaction mixture during the course of the reaction.

4. A process as claimed in any one of claims 1 to 3, in which the molar ratio of the compound of the general formula II to the alkylating agent is in the range from 1:1 to 5:1.

5. A process as claimed in any one of claims 1 to 4, in which the reaction is carried out under a pressure of up to 20 bars.

6. A process as claimed in any one of claims 1 to 5, in which the reaction temperature is in the range of from −30 to 80°C.

7. A process as claimed in any one of claims 1 to 6, in which the aniline compound of the general formula II, the alkylating agent and liquid HF are the only species present in the reaction mixture at the beginning of the reaction.

8. A process as claimed in any one of claims 1 to 7, in which a compound of formula I is prepared in which any alkyl group represented by $R^1$, $R^2$, $R^3$ or $R^4$ has up to 4 carbon atoms.

9. A process as claimed in claim 8, in which a compound of formula I is prepared in which $R^1$ represents a methyl group, $R^2$ represents an isopropyl group, and each of $R^3$ and $R^4$ represents a hydrogen atom.

10. A process as claimed in claim 9, in which the alkylating agent is propene.

11. A process as claimed in claim 1, carried out substantially as described in any one of Examples 1 to 4 herein.

12. A compound of the general formula I given in claim 1, whenever prepared by a process as claimed in any one of claims 1 to 11.

**Patentansprüche**

1. Verfahren zur Herstellung einer Verbindung der allgemeinen Formel

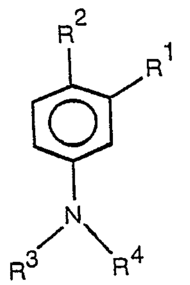

(I)

in der $R^1$ und $R^2$ jeweils unabhängig voneinander eine Alkylgruppe bedeuten und $R^3$ und $R^4$ unabhängig voneinander jeweils ein Wasserstoffatom oder eine Alkylgruppe bedeuten, umfassend die Umsetzung einer Verbindung der allgemeinen Formel

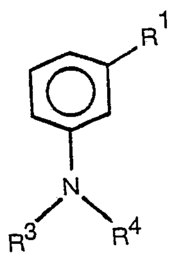

(II)

in der $R^1$, $R^3$ und $R^4$ die oben angegebene Bedeutung haban, mit einem Alkylierungsmittel, ausgewähalt aus einem Alken, einem Alkylhalogenid, einem Alkanol, einem Alkylester einer Carbonsäure und einem Dialkylether, dadurch gekennzeichnet, daß die Reaktion in flüssiger Phase in Gegenwart von Fluorwasserstoff durchgeführt wird.

2. Verfahren nach Anspruch 1, dadurch gekennzeichnet, daß das Alkylierungsmittel ein Alken oder Alkylhalogenid ist.

3. Verfahren nach Anspruch 1 oder 2, wobei das Alkylierungsmittel nach und nach während der Reaktion zu dem Reaktionsgemisch zugesetzt wird.

4. Verfahren nach einem der Ansprüche 1 bis 3, wobei das Molverhältnis der Verbindung der allgemeinen Formel II zu dem Alkylierungsmittel im Bereich von 1:1 bis 5:1 liegt.

5. Verfahren nach einem der Ansprüche 1 bis 4, wobei die Reaktion unter einem Druck von bis zu 20 bar durchgeführt wird.

6. Verfahren nach einem der Ansprüche 1 bis 5, wobei die Reaktionstemperatur im Bereich von −30 bis 80°C liegt.

7. Verfahren nach einem der Ansprüche 1 bis 6, wobei die Anilinverbindung der allgemeinen Formel II, das Alkylierungsmittel und das flüssige HF die einzigen zu Beginn der Umsetzung in dem Reaktionsgemisch vorhandenen Substanzen sind.

8. Verfahren nach einem der Ansprüche 1 bis 7, wobei eine Verbindung der Formel I hergestellt wird, bei der eine (jede) durch $R^1$, $R^2$, $R^3$ oder $R^4$ angegebene Alkylgruppe bis zu 4 Kohlenstoffatomen besitzt.

9. Verfahren nach Anspruch 8, wobei eine Verbindung der Formel I hergestellt wird, in der $R^1$ eine Methylgruppe, $R^2$ eine Isopropylgruppe und $R^3$ und $R^4$ jeweils ein Wasserstoffatom bedeuten.

10. Verfahren nach Anspruch 9, wobei das Alkylierungsmittel Propen ist.

11. Verfahren nach Anspruch 1 im wesentlichen durchgeführt wie in einem der Beispiele 1 bis 4 beschrieben.

12. Verbindung der allgemeinen Formel I nach Anspruch 1, soweit sie nach einem in einem der Ansprüche 1 bis 11 angegebenen Verfahren hergestellt worden ist.

**Revendications**

1. Procédé de préparation d'un composé de formule générale:

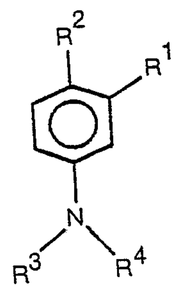

(I)

dans laquelle $R^1$ et $R^2$ représentent chacun indépendemment un groupe alkyle et $R^3$ et $R^4$ représentent chacun indépendemment un atome d'hydrogène ou un groupe alkyle, qui comprend la réaction d'un composé de formule générale

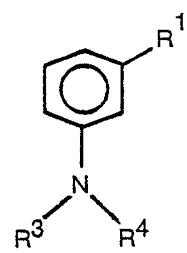

(II)

dans laquelle $R^1$, $R^3$ et $R^4$ ont les significations données ci-dessus, avec un agent d'alkylation choisi parmi un alcène, un halogénure d'alkyle, un alcanol, un ester alkylique d'un acide carboxylique ou un éther dialkylique, caractérisé en ce que la réaction est réalisée en phase liquide en présence de fluorure d'hydrogène.

2. Procédé selon la revendication 1, caractérisé en ce que l'agent d'alkylation est un alcène ou un halogénure d'alkyle.

3. Procédé selon l'une/des revendications 1 ou 2, caractérisé en ce que l'agent d'alkylation est ajouté graduellement au mélange réactionnel au cours de la réaction.

4. Procédé selon l'une quelconque des revendications 1 à 3, caractérisé en ce que le rapport molaire du composé de formule générale II à l'agent d'alkylation est compris entre 1:1 et 5:1.

5. Procédé selon l'une quelconque des revendications 1 à 4, caractérisé en ce que la réaction est réalisée sous une pression allant jusqu' à 2 MPa (20 bars).

6. Procédé selon l'une quelconque des revendications 1 à 5, caractérisé en ce que la température de la réaction est comprise entre −30 et 80°C.

7. Procédé selon l'une quelconque des revendications 1 à 6, caractérisé en ce que l'aniline de formule générale II, l'agent d'alkylation et le HF liquide sont les seules espèces présentes dans le mélange réactionnel au début de la réaction.

8. Procédé selon l'une quelconque des revendications 1 à 7, caractérisé en ce que l'on prépare un composé de formule I dans laquelle chacun des groupes alkyles représenté par $R^1$, $R^2$, $R^3$ ou $R^4$ a jusqu'à 4 atomes de carbone.

9. Procédé selon la revendication 8, caractérisé en ce que l'on prépare un composé de formule I dans laquelle $R^1$ représente un groupe méthyle, $R^2$ représente un groupe isopropyle, et $R^3$ et $R^4$ représentent chacun un atome d'hydrogène.

10. Procédé selon la revendication 9, caractérisé en ce que l'agent d'alkylation est le propène.

11. Procédé selon la revendication 1, mis en oeuvre pratiquement tel qu'il a été décrit dans l'un quelconque des exemples 1 à 4 cités ici.

12. Composé de formule générale I donné dans la revendication 1, préparé par le procédé revendiqué dans une quelconque des revendications 1 à 11.